# EUROPÄISCHE PATENTSCHRIFT

(11) **EP 1 404 305 B1**
(45) Veröffentlichungstag und Bekanntmachung des Hinweises auf die Patenterteilung: **12.11.2008**
(21) Anmeldenummer: 02758290.7
(22) Anmeldetag: 02.07.2002
(51) Int. Cl.: A61K 9/51, A61K 47/34

(54) **PHARMAKOLOGISCHE ZUBEREITUNG AUS EINEM NANOPARTIKULÄREN MESOMORPHEN POLYELEKTROLYT-LIPID-KOMPLEX UND MINDESTENS EINEM WIRKSTOFF**
PHARMACOLOGICAL PREPARATION MADE FROM A NANOPARTICULATE MESOMORPHOUS POLYELECTROLYTE LIPID COMPLEX AND AT LEAST ONE ACTIVE INGREDIENT
PREPARATION PHARMACOLOGIQUE OBTENUE A PARTIR D'UN COMPLEXE NANOPARTICULAIRE MESOMORPHE POLYELECTROLYTE-LIPIDE ET D'AU MOINS UNE SUBSTANCE ACTIVE

(30) Priorität: 05.07.2001 DE 10132669
(43) Veröffentlichungstag der Anmeldung: 07.04.2004
(73) Patentinhaber: Fraunhofer-Gesellschaft zur Förderung der angewandten Forschung e.V., 80686 München (DE); MAX-PLANCK-GESELLSCHAFT ZUR FÖRDERUNG DER WISSENSCHAFTEN E.V., 80539 München (DE)
(72) Erfinder: THÜNEMANN, Andreas, 14469 Potsdam (DE); GENERAL, Sascha, 14467 Potsdam (DE)
(74) Vertreter: Pfenning, Meinig & Partner GbR
(86) Internationale Anmeldenummer: PCT/EP2002/007255
(87) Internationale Veröffentlichungsnummer: WO 2003/004004

(56) Entgegenhaltungen:
- EP-A- 0 454 044
- WO-A-01/10413
- WO-A-99/47130
- US-A- 4 833 061

## Beschreibung

Die Erfindung betrifft eine pharmakologische Zubereitung aus einem nanopartikulären mesomorphen Polyelektrolyt-Lipid-Komplex und mindestens einem Wirkstoff. Der Polyelektrolyt-Lipid-Komplex weist dabei eine lamellare Struktur aus sich abwechselnden ionischen und nicht-ionischen Schichten auf, wobei der Wirkstoff in der nicht-ionischen Schicht eingelagert ist.

Mehrfach geladene makromolekulare Verbindungen bilden mit Ionen entgegengesetzter Ladung ionische Verbindungen, die oft in Abhängigkeit von der Ladungsverteilung, dem Molekulargewicht und der Hydrophobizität des Endprodukts aus wäßrigen Lösungen ausfallen. Dabei werden niedermolekulare Ionen gleicher Ladung durch die höhermolekulare Verbindung verdrängt. Bekannte Beispiele auf diesem Gebiet sind die Ausbildung von Gelen durch Zusammengeben von Alginatlösungen und Ca²⁺. Ebenso werden Proteinfällungen nach diesem Prinzip durchgeführt. Polyelektrolyt-Lipid-Komplexe können prinzipiell entweder aus einer makromolekularen, mehrfach geladenen Komponente einer Polarität und vielen niedermolekularen Ionen der anderen Polarität bestehen, oder aber aus zwei makromolekularen, jeweils mehrfach geladenen Partnern verschiedener Polarität aufgebaut sein. Aus der DE 40 13 110 A1 sind pharmazeutische Zubereitungen bekannt, die Polyelektrolyt-Lipid-Komplexe in mikropartikulärer Form enthalten.

In der modernen pharmazeutischen Technologie werden Formulierungen und Wirkstoffkombinationen immer wichtiger, die nicht nur den Wirkstoff in eine applizierbare Form bringen, sondern die gezielt die Bioverteilung, die Bioverfügbarkeit oder die Resorption des Arzneimittels beeinflussen. Hierdurch wird der Weg zu neuen therapeutischen diagnostischen Anwendungsbereichen eröffnet. Gleichzeitig kann auf diesem Wege auch die therapeutische Breite eines Wirkstoffes verbessert werden. Insbesondere partikuläre Systeme kleinsten Durchmessers (sog. Mikro- bzw. Nanopartikel) erweisen sich in jüngster Zeit als wichtige Applikationsform sowohl im oralen als auch im parenteralen Bereich an. (R.H. Müller et al., "Pharmazeutische Technologie: Moderne Arzneiformen", wissenschaftliche Verlagsgesellschaft mbH, 1997).

Aufgabe der vorliegenden Erfindung ist es daher, eine pharmakologische Zubereitung in einer einfach zu applizierenden Formulierung bereitzustellen, die es ermöglicht, die Bioverteilung, Bioverfügbarkeit und Resorption des Wirkstoffes zu steuern.

Diese Aufgabe wird durch die pharmakologische Zubereitung mit den Merkmalen des Anspruches 1 gelöst. Die weiteren abhängigen Ansprüche zeigen vorteilhafte Weiterbildungen auf.

Erfindungsgemäß wird eine pharmakologische Zubereitung aus einem nanopartikulären mesomorphen Polyelektrolyt-Lipid-Komplex und mindestens einem Wirkstoff bereitgestellt. Unter mesomorph wird hier ein flüssigkristallanaloger Ordnungszustand verstanden der eindeutig mittels kolloidanalytischen Methoden wie der Röntgenkleinwinkelstreuung nachgewiesen werden kann. Der Polyelektrolyt-Lipid-Komplex weist erfindungsgemäß eine lamellare Struktur aus sich abwechselnden ionischen und nicht-ionischen Schichten auf, wobei der mindestens eine Wirkstoff in der nicht-ionischen Schicht eingelagert ist. Über Lösungsgleichgewichte und Ladungswechselwirkung erfolgt dann in vivo ein langsames Dekomplexieren, was zur Auflösung des mesomorphen Komplexes unter Wirkstoffreisetzung führt. Die Wirkstoffreisetzung kann dabei über den pH-Wert des umgebenden Mediums beeinflußt werden.

Überraschenderweise konnte gezeigt werden, daß diese erfindungsgemäßen Polyelektrolyt-Lipid-Komplexe in nanopartikulärer Form eine sehr hohe chemische Stabilität und eine steuerbare Freisetzung des Wirkstoffs aufweisen. Dadurch ergibt sich ein einfacher Weg zur Immobilisierung chemisch empfindlicher Arzneistoffe. Des weiteren läßt sich die Kristallisation von Arzneistoffen durch diese Art der Formulierung in einfacher Weise unterdrücken.

In einer bevorzugten Ausführung ist der partikuläre Polyelektrolyt-Lipid-Komplex aus sphärischen ionischen und nicht-ionischen Schichten aufgebaut. Hierbei wechseln sich die einzelnen Schichten analog zu einer Zwiebelstruktur ab.

In einer weiteren Variante ist das Partikel aus planaren ionischen und nicht-ionischen Schichten aufgebaut. Hierbei wechseln sich die einzelnen Schichten in Form von Breitengraden oder Längengraden im Partikel ab.

In einer weiteren bevorzugten Ausführungsform weist das Partikel eine aus einem Polymer gebildete Schale auf, die das Partikel umgibt. Diese aus einem Polymer gebildete Schale kann bevorzugt aus Polyethylenoxid, Polyaminosäure, Polyethylenimin, Poly(dialyldimethylammoniumchlorid), Poly(N-methyl-4-vinylpyridinium-chlorid), Poly(N-ethyl-4-vinylpyridiniumchlorid), Poly(N-buthyl-4-vinylpyridiniumchlorid), Poly(4-vinyl-1-(3-sulphoprophylpyridiniumbetain), Poly(4-vinyl-1-carboxymethylpyridiniumbetain) ausgewählt sein.

Eine weitere Möglichkeit zur Steuerung der Freisetzung des mindestens einen Wirkstoffes erfolgt über die Partikelgröße. Dadurch wird es ermöglicht, daß anhand der Größe des Partikels die Zersetzungsdauer im Körper beeinflußt wird. Dabei liegt die Partikelgröße bevorzugt zwischen 10 und 500 nm, besonders bevorzugt zwischen 100 und 300 nm.

Für die Komplexbildung des Polyelektrolyt-Lipid-Komplexes werden bevorzugt natürlich vorkommende oder aus natürlichen Bausteinen bestehende biokompatible und bioabbaubare Polybasen eingesetzt. Die jeweiligen Gegenionen bestehen aus natürlichen oder synthetischen Lipiden. Als Lipide werden dabei bevorzugt Soja-Lecithin, Ei-Lecithin, gesättigte oder ungesättigte Fettsäuren und/oder deren Salze eingesetzt, z.B.

Dodecansäure und/oder Azelainsäure. Als Polybasen werden bevorzugt Polyethylenimin (PEI), dessen Derivate, Polyaminosäuren und/oder Chitosan eingesetzt. Ebenso ist es aber auch möglich, daß als Polybasen Blockcopolymere eingesetzt werden. Hierzu zählen beispielsweise ein Blockcopolymer aus einem Polyethylenoxid-Block und einem Polyaminosäure-Block oder ein Blockcopolymer aus einem Polyethylenoxid-Block und einem Polyethylenimin-Block. Als besonders bevorzugte Polyaminosäureblöcke werden die Homopolymere des Argenins, des Hystidins oder des Lysins eingesetzt.

Ein besonders bevorzugter Polyelektrolyt-Lipid-Komplex ist beispielsweise ein Komplex aus Polyethylenimin sowie Dodecansäure und/oder Azelainsäure sowie ein Komplex aus einem Blockcopolymer aus Polyethylenoxid und Polyethylenimin sowie Dodecansäure und/oder Azelainsäure.

Als eingelagerte Wirkstoffe kommen sämtliche aus der Pharmakologie gängigen Verbindungen in Frage. Hierzu zählen beispielsweise aktive Peptide, Proteine, Enzyme, Enzyminhibitoren, Antigene, Zytostatika und/oder Antibiotika.

In einer weiteren Variante kann die Partikeloberfläche chemisch modifiziert sein. Hierzu zählt beispielsweise die Kopplung mit Antikörpern oder DNA.

Anhand der folgenden Beispiele soll der erfindungsgemäße Gegenstand näher erläutert werden, ohne diesen auf diese Beispiele einzuschränken.

### Beispiel 1

**Polyethylenimin - Dodecansäure - Komplexe** 0,88 g (22 mmol) des PEI (M_{w} = 25000 g/mol) wurden in 15 mL einer Mischung aus 4 Teilen Ethanol und einem Teil Wasser gelöst. Unter Rühren und bei 65 °C wurden 11 mmol der Dodecansäure (C12), gelöst in Ethanol (65°C), über einen Zeitraum von 15 min dazugegeben. Nach weiteren 30 min Rühren wurde die Lösung in einer Teflonschale bei 40°C zu einem klaren Film getrocknet. Der Nachweis der vollständigen Komplexierung der Carbonsäurefunktionen wurde mit FT-IR geführt. Das Verschwinden der typischen IR-Carbonylbande bei 1690 cm⁻¹ im Komplex bewies das Fehlen von freien Säuregruppen. Mit polarisationsmikroskopischen Aufnahmen konnte die optische Anisotropie des Komplexes nachgewiesen werden. Die ausgebildeten Texturen wurden als Fächertexturen, typisch für lamellare Flüssigkristalle, identifiziert. DSC-Messungen zeigten einen Schmelzübergang der letzten 1.6 Methylengruppen der Alkylseitenketten der Dodecansäure im Komplex bei 4 °C. Das Fehlen von Kristallen im Komplex bei Raumtemperatur konnte mit Hilfe der Röntgenweitwinkelstreuung bestätigt werden. Nur eine amorphe Anordnung der Alkylketten mit einem mittleren Abstand der Ketten von 0.45 nm wurde gefunden. Die mesomorphe Struktur wurde mit Röntgenkleinwinkelstreuung untersucht. Eine lamellare Anordnung des Polyelektrolyten und der ionischen Kopfgruppe der Dodecansäure im Wechsel mit den Alkylresten der Carbonsäure wurde nachgewiesen. Dabei betrug der Lamellenabstand 2.9 nm und die Stapelordnung der Struktur über 600 nm senkrecht zur Lamellennormalen.

Die Modellarzneistoffe Coenzym Q₁₀ und Triiodthyronin wurden in Ethanol bzw. DMSO gelöst. Jeweils 17.7 mg (Triiodthyronin) und 25 mg (Q₁₀) wurden zu einer Lösung von 100 mg des Komplexes in THF gegeben. Die Mischungen wurden in einer Teflonschale getrocknet. Durch Weitwinkelröntgenstreuung und DSC-Messungen konnte wiederum die Abwesenheit von kristallinen Bereichen nachgewiesen werden. Selbst nach Lagerung der beladenen Komplexe von mehr als 150 Tagen unter Raumbedingungen konnten keine kristallinen Reflexe gefunden werden. Der Schmelzpunkt der Methylengruppen der Alkylreste sank durch die Beladung mit Arzneistoff auf - 8 °C, was auf eine Einlagerung der Substanzen in dieser Region der Lamelle hindeutet. Eine Phasentrennung von Komplex und Arzneistoffen konnte ausgeschlossen werden. Kleinwinkelröntgenstreuungen wiesen eine Anstieg des Lamellenabstandes durch die Einlagerung von etwa 0.3 nm nach.

Für die Präparation der Nanopartikel wurden 20 mg der mit 20 % [w/w] Q₁₀, bzw. 15 % [w/w] Triiodthyronin, beladenen Komplexe in 15 mL THF gelöst. Anschließend erfolgt eine Zugabe von 20 mL Wasser über einen Zeitraum von 10 min. Das THF wurde über 12 Stunden bei 50 °C und leichtem Rühren abgedampft. Anschließend wurde das mitverdampfte Wasser wieder ergänzt und die Dispersionen durch einen 800 nm Membranfilter filtriert. Es konnte, mittels NMR, kein THF in den Dispersionen nachgewiesen werden. Dabei lag die Nachweisgrenze dieser Methode bei unter 0,5 % [w/w]. Die Nanopartikel zeigten ein Zetapotential von + 50 mV und eine Größe die entsprechend der Ausgangskonzentration des Komplexes in der THF-Lösung im Bereich von 100 bis 200 nm lag. Dabei wurden die kleinsten Partikel aus einer 0.1 %-igen Lösung erhalten (hier beschrieben), die größten Partikel aus einer 1 %-igen Stammlösung. Mithilfe der Zetapotentialmessugen, AFM-Aufnahmen und TEM-Aufnahmen konnte gezeigt werden, dass die Partikel eine Kern-Schale-Morphologie aufweisen. Der Kern wird dabei aus einem nahezu stöchiometrischen Komlex des PEIs und der Dodecansäure gebildet, wobei die Schale aus nichtkomplexierten PEI-Ketten besteht; Stöchiometrie im Ansatz 2 (PEI) : 1 (Dodecansäure). Die nicht wasserlöslichen Modellarzneistoffe konnten in diesen Partikeln im Wasser stabil dispergiert werden. Es wurde kein Niederschlag gefunden. Durch Einlagerung der Fluoreszenzsonde Pyren (ebenfalls über den oben beschriebenen Weg) konnte eine Bestimmung der Polarität der Umgebung des Pyrens, und somit der Umgebung der eingelagerten Arzneistoffe ,durchgeführt werden. Dabei zeigte sich die Umgebung eher hydrophob, vergleichbar einer Lösung des Pyrens in Butanol. Eine solche Umgebung ist nur innerhalb des Komplexes und innerhalb der Alkylkettenschicht zu erwarten. Die Polarität innerhalb des Komplexes ist in den Partikeln im Vergleich zum wasserfreien Komplexfilm erhöht (dort vergleichbar mit einer Hexanlösung des Pyrens). Mithilfe von Röntgenkleinwinkelstreuungen konnte eine mesomorphe Struktur innerhalb der Partikel nachgewiesen werden. Geht man von einer lamellaren Struktur auch in den Partikeln aus, ergibt sich ein Anwachsen des Lamellenabstandes durch die Bildung der Nanopartikel auf 4.0 nm. Zusammen mit der Bestimmung der Mikropolarität innerhalb der Partikel lässt sich die Zunahme des Lamellenabstandes als Einlagerung von Wasser in die ionischen Schichten des Komplexes erklären. Im Vergleich zur mesomorphen Struktur in unbeladenen Partikeln aus dem Komplex ist der Lamellenabstand um die selben 0.3 nm erhöht, wie er durch die Einlagerung des Q₁₀ im Komplexfilm erhöht wurde.

Die Partikel wurden hinsichtlich ihrer Stabilität gegen den Einfluss von Fremdsalz, Verdünnung und pH-Wert Änderungen untersucht. Die Teilchengrößen der Partikel wurden mittel DLS bei Veränderung der Kochsalzkonzentration des wässrigen Mediums untersucht. Dabei zeigte sich, dass die Partikel in ihrer Größe bis zu einer NaCl-Konzentration von 0.3 mol/L über Tage stabil waren. Bei Verdünnung der Partikeldispersion wurde durch online Bestimmung der Oberflächenspannung, Leitfähigkeit und Trübung nachgewiesen, dass die Partikel bis zu einer Konzentration von 1 mg/L stabil in Größe und Zusammensetzung blieben. Bei Änderungen des pH-Werts des wässrigen Mediums wurde gefunden, dass die Partikel unterhalb eines pH-Wertes von 4.2 und oberhalb pH 8 nicht stabil blieben. Unterhalb eines pH-Wertes von 4.2 trat ein Niederschlag auf, der mit Hilfe der DSC als reine Dodecansäure identifiziert wurde. Es konnten bei diesem pH-Wert keine Partikel nachgewiesen werden, d.h. der Komplex der den Kern der Partikel bildete, hatte sich getrennt, wobei die bei diesem pH-Wert wasserunlösliche Dodecansäure ausfiel und das PEI molekular in Lösung blieb. Bei Erhöhung des pH-Wertes über pH 8 hinaus konnten ebenfalls keine Partikel mehr beobachtet werden. Es wurde kein Niederschlag gefunden. Der Komplex hat sich ebenfalls getrennt, wobei die verbliebenen Komponenten bei diesem pH-Wert wasserlöslich sind. Die Schlussfolgerung wurden durch Untersuchungen der Polarität der Umgebung durch die Einlagerung der Fluoreszenzsonde Pyren bestätigt. In dem Stabilitätsfenster der Partikel zwischen pH 4.2 und pH 8 war die Polarität vergleichbar einer Butanol-lösung des Pyrens. Bei Überschreitung der Werte wurde die Umgebung des Pyrens polarer und erreichte die Werte für eine wässrige Lösung des Pyrens.

### Beispiel 2

### Polyaminosäuren - Dodecansäure - Komplexe

Zur Partikelpräparation wurden 40 mL einer 2 mmol/L (bezogen auf die Ladungen) Lösung der Polyaminosäuren Poly- L -Histidin (PLH), Poly- L -Arginin (PLA) und Poly-L-Lysin (PLL) in Wasser vorgelegt und mit 4 mL einer 10 mmol/L (auf pH 9,5 eingestellten) wässrigen Lösung der Dodecansäure langsam und unter Rühren versetzt. Anschließend wurde die Dispersion durch einen 800 nm Membranfilter filtriert.
Zur Einlagerung des Q10 und des Pyrens wurden 3,4 mg, bzw. 0,3 mg, in 8 mL THF gelöst und mit der Dodecansäurelösung vor Komplexbildung versetzt. Anschließend wurde das THF bei 50 °C über einen Zeitraum von 12 Stunden verdampft. Auch hier konnten nach Verdampfen des THF's keine Spuren des Lösungsmittels mittels NMR nachgewiesen werden. Die Partikelgrößen wurden über DLS für alle Polyaminosäuren zu 180 - 205 nm bestimmt. Das Zetapotential der Partikel waren abhängig von verwendeten Polyaminosäure + 42 mV (PLH-Dodecansäure), +59 mV (PLL-C12) und + 67 mV (PLA-C12). Zusammen mit AFM-Aufnahmen der Partikel konnte eine Kern-Schale-Morphologie nachgewiesen werden. Der Kern besteht aus nahezu stöchiometrischem Komplex, die Schale aus nichtkomplexierten Polyaminosäureketten. Über CD-Messungen konnte gezeigt werden, dass die Polyaminosäure im Komplex, also im Kern der Partikel, in einer geordneten Sekundärstruktur vorliegt, wohingegen die stabilisierenden nichtkomplexierten Ketten au der Oberfläche der Partikel in einer ungeordneten Knäuel Konformation auftreten. Für die PLL und PLA-Dodecansäure Komplexe innerhalb der Partikel konnte eine dominierende □-Helix Konformation nachgewiesen werden, für den PLH-Dodecansäure Komplex eine □-Faltblatt Konformation. Mittels analytischer Ultrazentrifugation wurde gezeigt, dass eingelagertes Q₁₀ mit dem selben Sedimentationkoeffizienten sedimentiert wie die Nanopartikel, was den Schluss zulässt, dass das Q₁₀ in den Partikeln dispergiert ist. Auch nach Lagerung der wässrigen Dispersion der beladenen Partikel über mehr als 20 Tage konnte kein Niederschlag des wasserunlöslichen Q₁₀ gefunden werden. Die Teilchengrößen der Partikel wurden mittel DLS bei Veränderung der Kochsalzkonzentration des wässrigen Mediums untersucht. Dabei zeigte sich, dass die Partikel in ihrer Größe bis zu einer NaCl-Konzentration von mindestens 0.3 mol/L über Tage stabil waren. Die Stabilität der Partikel wurde bei Änderung des pH-Wertes des wässrigen Mediums hinsichtlich Zetapotential und Teilchengröße untersucht. Entsprechend der pKs-Werte der Polyaminosäuren, kam es bei basischen pH-Werten zum Abfall der Zetapotentiale und somit zur Destabilisierung der Partikel und zum Niederschlag. Das trat für die PLH-C12 Partikel bei einem pH-Wert von größer als 6.8, bei den PLL-C12 Partikeln bei einem pH-Wert von größer als 9.8 und bei den PLA-C12 Partikeln bei einem pH-Wert von größer als 11. Damit ist die Pärtikeldestabilisierung und Komplexauflösung über die Basizität der Polyaminosäuren einstellbar. Bei einem pH-Wert von kleiner 4.3 kam es bei allen Polyaminosäuren zur Auftrennung des Komplexes und zum Niederschlag der reinen Dodecansäure, wobei die Polyaminosäuren in Lösung blieben. Die Komplexauflösung wurde durch die Fluoreszenzsonde Pyren untersucht. In den jeweiligen Stabilitätsfenstern der Komplexpartikel ist die Polarität der Umgebung des eingelagerten Pyrens eher unpolar, beim überschreiten der Stabilitätsgrenzen nähert sich die Polarität den Werten für eine wässrige Umgebung des Pyrens an, das Pyren wird in das wässrige Medium freigesetzt.

### Beispiel 3

### PEO-block-PEI - Dodecansäure- Komplexe

Jeweils 100 mg der 3 verschiedenen Polyelektrolytblockcopolymere (entsprechen 1.14* 10⁻⁴ mol Aminfunktionen des PEO-*b*-PEI_{cy}, 1.95*10⁻⁴ mol Aminfunktionen des PEO-*b*-PEIₗᵢ und 2.8*10⁻⁴ mol Aminfunktionen des PEO-*b*-PEI_{br}) wurden in 60 °C heißem Ethanol gelöst.

Anschließend wurden 0,5 Äquivalente der Dodecansäure als 1%-ige Lösung in heißem Ethanol dazugegeben. Die klare Lösung wurde weitere 30 min gerührt und anschließend in eine Teflonschale gegossen und getrocknet. Durch Zugabe der entsprechenden Mengen an Q₁₀ und Triiodthyronin als Lösung in THF bzw. DMSO zur Lösung der Dodecansäure vor Komplexierung wurde die Einlagerung der Modellarzneistoffe erreicht. Die beladenen Komplexe wurden anschließend getrocknet. Eine makroskopische Orientierung des PEO-PEIₗᵢ - C12 - Komplexes wurde durch Lagerung zwischen zwei Teflonfolien unter einem mechanischen Druck von 10 N/cm² über 12 Stunden erreicht. Durch FT-IR Messungen konnte eine vollständige Komplexierung der Dodecansäure nachgewiesen werden. DSC-Messungen und Röntgenweitwinkelstreuungen zeigten, dass die Kristallisation der PEO-Ketten durch Komplexierung des PEI-Blockes nicht verhindert wurde und dass der PEI-C12 Komplex in einer amorphen Form vorlag. Die eingelagerten Modellarzneistoffe waren dabei amorph in der Alkylschicht der Dodecansäure dispergiert. Durch Röntgenkleinwinkelstreuung wurde eine Strukturhierarchie nachgewiesen. Dabei wird eine Lamelle von etwa 15 nm Dicke durch das Blockcopolymer erzeugt und eine, senkrecht dazu stehende, Lamelle durch den Komplex der PEI-Blöcke mit der Dodekansäure. Die Komplexlamelle ist mit etwa 3 nm Lamellenabstand 5 mal kleiner als die große Lamelle. Die Nanopartikel wurden durch kontrollierte Zugabe von Lösen von 20 mL Wasser zu 20 mg des entsprechenden Komplexes in präpariert. Nach 30 minütigem Rühren bei 35 °C wurde die Dispersion durch einen 800 nm Membranfilter filtriert. Das Zetapotential der so gebildeten Partikel war 0 mV. Die Teilchengröße, bestimmt durch DLS, lag bei 200 nm. TEM-Aufnahmen zeigten eine Kern-Schale-Morphologie der Partikel, wobei die Kerne aus den Komplexen der PEI-Blöcke und der Dodecansäure bestanden und die Schale aus PEO-Ketten. Die-Partieklmorphologie konnte durch die Variation der PEI-Block Architektur von stark elongiert über elongiert bis zu sphärisch verändert werden.

## Patentansprüche

1. Pharmakologische Zubereitung aus einem nanopartikulären mesomorphen Polyelektrolyt-Lipid-Komplex und mindestens einem Wirkstoff, wobei der Polyelektrolyt-Lipid-Komplex eine lamellare Struktur aus sich abwechselnden ionischen und nicht-ionischen Schichten aufweist und der mindestens eine Wirkstoff in der nicht-ionischen Schicht eingelagert ist und dessen Freisetzung pH-abhängig steuerbar ist.

2. Zubereitung nach Anspruch 1,
**dadurch gekennzeichnet, dass** das Partikel aus sphärischen ionischen und nicht-ionischen Schichten aufgebaut ist.

3. Zubereitung nach Anspruch 1,
**dadurch gekennzeichnet, dass** das Partikel aus planaren ionischen und nicht-ionischen Schichten aufgebaut ist.

4. Zubereitung nach mindestens einem der Ansprüche 1 bis 3,
**dadurch gekennzeichnet, dass** das Partikel von einer aus einem Polymer gebildeten Schale umgeben ist.

5. Zubereitung nach Anspruch 4,
**dadurch gekennzeichnet, dass** die Schale aus Polyethylenoxid, Polyaminosäure, Polyethylenimin, Poly(diallyldimethylammoniumchlorid), Poly(N-methyl-4-vinylpyridiniumchlorid), Poly(N-ethyl-4-vinylpyridiniumchlorid), Poly(N-butyl-4-vinylpyridiniumchlorid), Poly(4-vinyl-1-(3-sulfopropyl)pyridiniumbetain), Poly(4-vinyl-1-carboxymethylpyridiniumbetain).

6. Zubereitung nach mindestens einem der Ansprüche 1 bis 5,
**dadurch gekennzeichnet, dass** die Freisetzung des mindestens einen Wirkstoffs über die Partikelgröße steuerbar ist.

7. Zubereitung nach mindestens einem der Ansprüche 1 bis 6,
**dadurch gekennzeichnet, dass** die Partikelgröße zwischen 10 und 500 nm, bevorzugt zwischen 100 und 300 nm liegt.

8. Zubereitung nach mindestens einem der Ansprüche 1 bis 7,
**dadurch gekennzeichnet, dass** der Polyelektrolyt-Lipid-Komplex unter Verwendung von Sojalecithin, Eilecithin, gesättigten oder ungesättigten Fettsäuren und/oder deren Salze als Lipid gebildet ist.

9. Zubereitung nach mindestens einem der Ansprüche 1 bis 8,
**dadurch gekennzeichnet, dass** der Polyelektrolyt-Lipid-Komplex unter Verwendung von Polyethylenimin (PEI), dessen Derivaten, einer Polyaminosäure und/oder Chitosan als Polybase gebildet ist.

10. Zubereitung nach mindestens einem der Ansprüche 1 bis 9,
**dadurch gekennzeichnet, dass** der Polyelektrolyt-Lipid-Komplex unter Verwendung von Blockcopolymeren, z.B. aus einem Polyethylenoxid-Block und einem Polyaminosäure-Block oder einem Polyethylenoxid-Block und einem Polyethylenimin-Block als Polybase gebildet ist.

11. Zubereitung nach mindestens einem der Ansprüche 1 bis 10,
**dadurch gekennzeichnet, dass** der Polyelektrolyt-Lipid-Komplex aus Polyethylenimin sowie Dodecansäure und/oder Azelainsäure gebildet ist.

12. Zubereitung nach mindestens einem der Ansprüche 1 bis 10,
**dadurch gekennzeichnet, dass** der Polyelektrolyt-Lipid-Komplex aus einem Blockcopolymer aus Polyethylenoxid und Polyethylenimin sowie Dodecansäure und/oder Azelainsäure gebildet ist.

13. Zubereitung nach mindestens einem der Ansprüche 1 bis 12,
dass als Wirkstoffe aktive Peptide, Proteine, Enzyme, Enzyminhibitoren, Antigene, Cytostatika und/oder Antibiotika enthalten sind.

14. Zubereitung nach mindestens einem der Ansprüche 1 bis 13,
**dadurch gekennzeichnet, dass** die Partikeloberfläche chemisch, z.B. durch Kopplung mit Antikörpern oder DNA modifiziert ist.

## Claims

1. Pharmacological preparation composed of a nanoparticulate mesomorphic polyelectrolyte-lipid complex and at least one active compound, wherein the polyelectrolyte-lipid complex has a lamellar structure of alternating ionic and nonionic layers and the at least one active compound is embedded in the nonionic layer and the release thereof can be controlled as a function of the pH.

2. Preparation according to claim 1,
**characterized in that** the particle is built up from spherical ionic and nonionic layers.

3. Preparation according to claim 1,
**characterized in that** the particle is built up from planar ionic and nonionic layers.

4. Preparation according to at least one of claims 1 to 3,
**characterized in that** the particle is enclosed by a shell formed from a polymer.

5. Preparation according to claim 4,
**characterized in that** the shell is selected from polyethylene oxide, polyamino acid, polyethylenimine, poly(diallyldimethylammonium chloride), poly(N-methyl-4-vinylpyridinium chloride), poly(N-ethyl-4-vinylpyridinium chloride), poly(N-butyl-4-vinylpyridinium chloride), poly(4-vinyl-1-(3-sulfopropyl)pyridinium betaine) or poly (4-vinyl-1-carboxymethylpyridinium betaine).

6. Preparation according to at least one of claims 1 to 5,
**characterized in that** the release of the at least one active compound can be controlled via the particle size.

7. Preparation according to at least one of claims 1 to 6,
**characterized in that** the particle size is between 10 and 500 nm, preferably between 100 and 300 nm.

8. Preparation according to at least one of claims 1 to 7,
**characterized in that** the polyelectrolyte-lipid complex is formed using soya lecithin, egg lecithin, saturated or unsaturated fatty acids and/or salts thereof as the lipid.

9. Preparation according to at least one of claims 1 to 8,
**characterized in that** the polyelectrolyte-lipid complex is formed using polyethylenimine (PEI), derivatives thereof, a polyamino acid and/or chitosan as the polybase.

10. Preparation according to at least one of claims 1 to 9,
**characterized in that** the polyelectrolyte-lipid complex is formed using block copolymers, e.g. of a polyethylene oxide block and a polyamino acid block or a polyethylene oxide block and a polyethylenimine block as the polybase.

11. Preparation according to at least one of claims 1 to 10,
**characterized in that** the polyelectrolyte-lipid complex is formed from polyethylenimine and dodecanoic acid and/or azelaic acid.

12. Preparation according to at least one of claims 1 to 10,
**characterized in that** the polyelectrolyte-lipid complex is formed from a block copolymer of polyethylene oxide and polyethylenimine as well as dodecanoic acid and/or azelaic acid.

13. Preparation according to at least one of claims 1 to 12,
**characterized in that** it contains active peptides, proteins, enzymes, enzyme inhibitors, antigens, cytostatics and/or antibiotics as active compounds.

14. Preparation according to at least one of claims 1 to 13,
**characterized in that** the particle surface is modified chemically, e.g. by coupling with antibodies or DNA.

## Revendications

1. Composition pharmacologique constituée d'un complexe polyélectrolyte-lipide mésomorphe nanoparticulaire et d'au moins un principe actif, où le complexe polyélectrolyte-lipide présente une structure lamellaire constituée de couches ioniques et non ioniques en alternance et où le au moins un principe actif est incrusté dans la couche non ionique et sa libération peut être régulée en fonction du pH.

2. Composition selon la revendication 1, **caractérisée en ce que** la particule est constituée de couches sphériques ioniques et non ioniques.

3. Composition selon la revendication 1, **caractérisée en ce que** la particule est constituée de couches planes ioniques et non ioniques.

4. Composition selon au moins une des revendications 1 à 3, **caractérisée en ce que** la particule est entourée d'une coque formée à partir d'un polymère.

5. Composition selon la revendication 4, **caractérisée en ce que** la coque consiste d'oxyde de polyéthylène, d'acide polyaminé, de polyéthylène-imine, de poly(chlorure diallyldiméthylammonium), de poly(chlorure de N-méthyl-4-vinylpyridinium), de poly(chlorure de N-éthyl-4-vinylpyridinium), de poly(chlorure de N-butyl-4-vinylpyridinium), de poly(bétaïne de 4-vinyl-l-(3-sulfopropyl)pyridinium), de poly(bétaïne de 4-vinyl-1 -carboxyméthylpyridinium).

6. Composition selon au moins une des revendications 1 à 5, **caractérisée en ce que** la libération du au moins un principe actif peut être régulée par le biais de la taille des particules.

7. Composition selon au moins une des revendications 1 à 6, **caractérisée en ce que** la taille des particules se situe entre 10 et 500 nm, de préférence entre 100 et 300 nm.

8. Composition selon au moins une des revendications 1 à 7, **caractérisée en ce que** le complexe polyélectrolyte-lipide est formé en utilisant la lécithine de soja, la lécithine d'oeuf, des acides gras saturés ou insaturés et/ou leurs sels à titre de lipides.

9. Composition selon au moins une des revendications 1 à 8, **caractérisée en ce que** le complexe polyélectrolyte-lipide est formé en utilisant la polyéthylène-imine (PEI), ses dérivés, un acide polyaminé et/ou du chitosane à titre de polybase.

10. Composition selon au moins une des revendications 1 à 9, **caractérisée en ce que** le complexe polyélectrolyte-lipide est formé en utilisant des copolymères blocs, par exemple constitués d'un bloc d'oxyde de polyéthylène et d'un bloc d'acide polyaminé ou d'un bloc d'oxyde de polyéthylène et d'un bloc de polyéthylène-imine à titre de polybase.

11. Composition selon au moins une des revendications 1 à 10, **caractérisée en ce que** le complexe polyélectrolyte-lipide est formé à partir de polyéthylène-imine ainsi que d'acide dodécanoïque et/ou d'acide azélaïque.

12. Composition selon au moins une des revendications 1 à 10, **caractérisée en ce que** le complexe polyélectrolyte-lipide est formé à partir d'un copolymère à bloc d'oxyde de polyéthylène et de polyéthylène-imine ainsi que d'acide dodécanoïque et/ou d'acide azélaïque.

13. Composition selon au moins une des revendications 1 à 12, **caractérisée en ce que** des peptides, des protéines, des enzymes, des inhibiteurs enzymatiques, des antigènes, des cytostatiques et/ou des antibiotiques, actifs, sont présents à titre de principes actifs.

14. Composition selon au moins une des revendications 1 à 13, **caractérisée en ce que** la surface des particules est modifiée chimiquement, par exemple par couplage avec des anticorps ou de l'ADN.
